# DEMANDE DE BREVET EUROPEEN

(11) **EP 4 821 479 A1**
(43) Date de publication de la demande: **14.10.2026**
(21) Numéro de dépôt: 26170782.2
(22) Date de dépôt: 03.04.2026
(51) Int. Cl.: A61K 8/06, A61K 8/368, A61K 8/92, A61K 8/9789, A61Q 5/00, A61Q 19/00, A61Q 19/02, A61Q 19/08, A61K 36/22, A61P 17/12, A61P 17/00, B01D 11/00

(54) **UTILISATION COSMÉTIQUE D'UN EXTRAIT ÉTHANOLIQUE DE COQUES DE NOIX DE CAJOU EN TANT QU AGENT EXFOLIANT**

(30) Priorité: 07.04.2025 FR 2503636
(71) Demandeur: Ephyla, 56190 Arzal (FR)
(72) Inventeur: BOURGETEAU, Vincent, 56130 FEREL (FR)
(74) Mandataire: Eveillard, Sophie

(57) **Abrégé**

Utilisation d'un extrait de coques de noix de cajou susceptible d'être obtenu par un procédé de préparation comprenant au moins les étapes suivantes, dans cet ordre : fournir des coques de noix de cajou, broyer les coques de noix de cajou pour obtenir une taille moyenne des particules inférieure ou égale 5 mm, procéder à l'extraction des coques de noix de cajou au moyen d'éthanol à 96° à une température comprise entre 20°C et 45°C, filtrer, récupérer le filtrat, évaporer l'éthanol, récupérer l'extrait obtenu ou d'une composition comprenant cet extrait en tant qu'agent exfoliant de l'épiderme.

## Description

[La présente invention se rapporte au domaine de l'exfoliation de l'épiderme, encore appelée « épidermabrasion ». Plus précisément l'invention concerne un extrait de coques de noix de cajou ainsi que son utilisation en cosmétique, en application sur la peau, en tant qu'agent exfoliant de l'épiderme.

La peau est la principale barrière de protection du corps humain à l'égard des agressions extérieures telles que la pollution atmosphérique, les variations climatiques et le rayonnement UV.

La peau est constituée de trois couches : l'épiderme, le derme et l'hypoderme. L'épiderme, qui est la couche superficielle en contact avec l'extérieur, est plus particulièrement concerné par les interactions avec le milieu extérieur.

L'épiderme est recouvert par un film hydrolipidique et est composé de quatre couches : la couche cornée, la couche granuleuse, la couche épineuse et la couche basale. L'épiderme est normalement constitué de quatre types de cellules : les kératinocytes qui représentent 80% de l'ensemble de ses cellules, les 20% restant étant composés des mélanocytes, des cellules de Langerhans ou cellules immunocompétentes et des cellules de Merkel, ces trois groupes de cellules étant dispersées entre les kératinocytes.

La peau se renouvelle et se régénère continuellement *via* un processus appelé le renouvellement cellulaire. Le renouvellement cellulaire de l'épiderme permet d'éliminer les cellules mortes de la peau et de les remplacer par des cellules nouvellement formées. Outre de conférer à la peau un aspect jeune et frais, le renouvellement cellulaire permet aussi de conserver une peau lisse, saine et uniforme.

Il existe différentes techniques pour stimuler le renouvellement cellulaire tels que les traitements au laser, les traitements mécaniques de (micro)dermabrasion ou les peelings chimiques encore appelés « dermabrasions chimiques ».

Les acides de fruits (AHA) sont les principaux actifs utilisés dans le domaine cosmétique pour mettre en œuvre des peelings chimiques.

Le problème posé à l'origine de la présente invention était de proposer de nouvelles compositions cosmétiques à base de produits naturels présentant des propriétés exfoliantes de l'épiderme.

L'inventeur a montré qu'une solution à ce problème technique pouvait être apportée au moyen d'un extrait particulier de coques de noix de cajou.

Cet extrait de noix de cajou, ou *« Anacardium occidentale L.* », particulier permet de mettre en œuvre une abrasion ou exfoliation contrôlée des couches supérieures de l'épiderme.

Contrairement aux compositions de peeling d'acide, l'extrait de coques de noix de cajou présente l'avantage de ne pas requérir d'être placé en milieu acide pour être actif même s'il est toujours efficace à pH acide. En conséquence, l'utilisation de l'extrait de cajou selon la présente invention permet de limiter voire d'éviter les irritations et l'inconfort des picotements ou de l'abrasion. Les peaux sensibles et réactives trouvent ainsi une solution de gommage qui agit en délicatesse de manière à préserver le confort du soin.

### Résumé de l'invention

Selon un premier aspect, la présente invention vise l'utilisation cosmétique, non thérapeutique, par voie topique d'un extrait de coques de noix de cajou ou *« Anacardium occidentale L.* » en tant qu'agent exfoliant de l'épiderme, ledit extrait de coques de noix de cajou ou *« Anacardium occidentale L.* » étant susceptible d'être obtenu par un procédé de préparation comprenant au moins les étapes suivantes, dans cet ordre :
- fournir des coques de noix de cajou,
- broyer les coques de noix de cajou pour obtenir une taille moyenne des particules inférieure ou égale 5 mm,
- procéder à l'extraction des coques de noix de cajou au moyen d'éthanol à 96° à une température comprise entre 20°C et 45°C,
- filtrer,
- récupérer le filtrat,
- évaporer l'éthanol,
- récupérer l'extrait obtenu.

Selon un deuxième aspect, la présente invention vise l'utilisation cosmétique, non thérapeutique, par voie topique d'une composition comprenant de 0,1 à 49,9 % en poids dudit extrait de coques de noix cajou en tant qu'agent exfoliant de l'épiderme.

Selon un troisième aspect, la présente invention vise l'utilisation cosmétique, non thérapeutique, par voie topique de l'extrait de coques de noix de cajou ou de la composition comprenant l'extrait de coques de noix de cajou en tant qu'agent exfoliant de l'épiderme pour effacer les taches d'hyperpigmentation de la peau et/ou améliorer l'éclat de la peau et/ou pour conférer à la peau un aspect plus lisse, plus clair et/ou plus uniforme.

Selon quatrième aspect, la présente invention vise un procédé de traitement cosmétique, non thérapeutique d'exfoliation de l'épiderme comprenant au moins une étape d'application sur la peau dudit extrait de coques de noix de cajou ou de ladite composition selon l'invention comprenant ledit extrait de coques de noix de cajou.

En débarrassant la peau de ses vieilles cellules, dont celles pigmentées, l'extrait et la composition utilisés selon la présente invention favorisent le renouvellement cellulaire. Or le renouvellement cellulaire contribue à atténuer les décolorations de la peau telles que les taches de vieillesse et les taches de soleil, ce qui confère à la peau un teint plus uniforme.

En vieillissant, le renouvellement cellulaire naturel de la peau ralentit. En stimulant ce processus, les ridules et les rides s'atténuent, ce qui rajeunit l'apparence de la peau.

Les compositions cosmétiques selon l'invention sont en outre stables, non irritantes, non toxiques, non allergisantes pour la peau.

D'autres aspects, avantages, propriétés de la présente invention sont présentés dans la description et les exemples qui suivent.

### Description détaillée

### Définitions

Dans le présent texte, sauf indications contraires spécifiques, les pourcentages sont exprimés en poids d'une composition de référence.

Dans le présent texte, les intervalles sont définis de façon abrégée afin d'éviter de décrire chacune des valeurs et toutes les valeurs de cet intervalle, cependant toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2 ; 0,3 ; 0,4 ; 0,5 ; 0,6 ; 0,7 ; 0,8 ; 0,9 et tous les intervalles intermédiaires compris dans 0,1 à 1,0, tels que 0,2 à 0,5 ; 0,2 à 0,8 ; 0,7 à 1,0... Sauf indications contraires, un intervalle défini comme étant « compris entre la valeur A et la valeur B » inclut les valeurs A et B et est donc équivalent à un intervalle « allant de la valeur A à la valeur B » , l'expression « au moins » comprend la valeur énoncée après, par exemple, «au moins 5 % » doit être compris comme comprenant également « 5 % », l'expression « un maximum de » comprend la valeur énoncée après, par exemple, « un maximum de 5 % » doit être compris comme comprenant également « 5 % ».

En outre, dans le présent texte, les valeurs mesurables, telles qu'une quantité, doivent être comprises comme comprenant les écarts types qui peuvent être facilement déterminés par l'homme du métier dans le domaine technique de référence.

Par « peau » on entend l'épiderme du visage ou du corps ou du cuir chevelu.

Dans le présent texte, les expressions « effet exfoliant », « effet peeling », « effet épidermabrasion », « effet gommage » sont utilisées de manière équivalente.

Les compositions selon la présente invention sont des compositions cosmétiques, non thérapeutiques. Les compositions cosmétiques sont destinées à être appliquées sur les peaux saines pour traiter l'épiderme. Elles sont conformes au règlement CE 1223/2009.

L'acide anacardique est un mélange de plusieurs molécules présentant la structure suivante : un noyau correspondant à l'acide salicylique comprenant, un radical -CH₂-R, le radical R étant un radical hydrocarboné en C₁₄ comprenant de 0 à 3 insaturations. En particulier l'acide anacardique comprend les quatre dérivés suivants d'acide 6-alkylsalicylique de formule I :

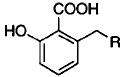

| nombre de C, nombre d'insaturations (sur le radical CH2R) | Nom |
|---|---|
| C15, 0 | Acide 6-n-pentadecylsalicylique |
| C15, 1 | Acide 6- [8(Z)-n-pentadecenyl] salicylique |
| C15, 2 | Acide 6- [8(Z), 11(Z)-n-pentadecadienyl] salicylique |
| C15, 3 | Acide 6- [8(Z), 11(Z), 14(Z)-n-pentadecatrienyl] salicylique |

En particulier l'acide anacardique peut être obtenu à partir de coques de noix de cajou.

### Extrait de coques de noix de cajou

L'extrait de coques de noix de cajou ou *Anacardium occidentale* L. utilisé dans la présente invention est susceptible d'être obtenu par un procédé de préparation comprenant au moins les étapes suivantes, dans cet ordre :
- fournir des coques de noix de cajou,
- broyer les coques de noix de cajou pour obtenir une taille moyenne des particules inférieure ou égale 5 mm,
- procéder à l'extraction des coques de noix de cajou au moyen d'éthanol à 96° à une température comprise entre 20°C et 45°C,
- filtrer,
- récupérer le filtrat,
- évaporer l'éthanol,
- récupérer l'extrait obtenu.

En particulier cet extrait de coques de noix cajou comprend de 60 à 95 % en poids d'acide anacardique par rapport au poids total de l'extrait. Il s'agit donc d'un extrait concentré en acide anacardique.

En outre cet extrait de coques de noix cajou comprend de 50 à 90 % en poids d'acide 6- [8(Z)-n-pentadecenyl] salicylique par rapport au poids total de l'extrait.

### Procédé de préparation de l'extrait

Comme déjà mentionné, l'extrait peut être préparé selon un procédé comprenant au moins les étapes suivantes, dans cet ordre :
- fournir des coques de noix de cajou,
- broyer les coques de noix de cajou pour obtenir une taille moyenne des particules inférieure ou égale 5 mm,
- procéder à l'extraction des coques de noix de cajou au moyen d'éthanol à 96° à une température comprise entre 20°C et 45°C,
- filtrer,
- récupérer le filtrat,
- évaporer l'éthanol,
- récupérer l'extrait obtenu.

### Composition

La présente invention vise aussi l'utilisation cosmétique, non thérapeutique d'une composition comprenant de 0,1 à 49,9 % en poids dudit extrait de coques de noix cajou selon l'invention par rapport au poids total de la composition.

Selon une première variante, la composition selon l'invention est une composition essentiellement huileuse.

Selon cette première variante, ladite composition comprend de 0,1 à 99,9% en poids d'une phase huileuse végétale comprenant au moins une huile végétale et/ou au moins un ester de corps gras végétal par rapport au poids total de la composition.

Selon un mode de réalisation préféré, la phase huileuse végétale comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de chanvre, l'huile de nigelle, l'huile de figue de barbarie, l'huile de macadamia, l'huile de soja, l'huile de palme et de palmier, l'huile de Tamanu, l'huile de Kukui (ou bancoulier), l'huile de sésame, l'huile de lin, l'huile de noix, l'huile de noisette, l'huile d'amande, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan et les triglycérides, notamment d'acides caprylique/caprique.

Selon une deuxième variante particulière, préférée, la composition selon l'invention comprend une émulsion de Pickering H/E contenant une phase aqueuse, une phase huileuse végétale et un phyllosilicate organomodifié.

Les émulsions de Pickering sont des émulsions stabilisées par des particules solides. Lors de la préparation de l'émulsion, lesdites particules solides se positionnent à l'interface entre la phase aqueuse et la phase huileuse.

Les émulsions de Pickering utilisées selon la présente invention sont des émulsions huile dans eau, c'est-à-dire H/E, stabilisées par une argile particulière qui est un phyllosilicate naturel modifié.

Avantageusement, la phase aqueuse de l'émulsion de Pickering est présente en une quantité allant de 43% à 93%, de préférence 55% à 70% et de manière préférée 58% à 65% en poids par rapport au poids total de la composition.

Selon un mode de réalisation préféré, le phyllosilicate naturel modifié comprend un phyllosilicate choisi dans le groupe constitué par les vermiculites et les smectites. De préférence, le phyllosilicate peut être choisi dans le groupe constitué par les montmorillonites, bentonites, nonytronites, beidellites, volkonskoites, hectorites, saponites, sauconites, sobockites, stevensites, svinfordites, de préférence ce sont des phyllosilicates de sodium, de potassium, de calcium, ou de leurs mélanges. De manière plus préférée, le phyllosilicate est choisi dans le groupe constitué par l'hectorite, la montmorillonite, la bentonite ou leurs mélanges.

Selon un mode de réalisation préféré, le phyllosilicate naturel modifié comprend un composé organique choisi dans le groupe constitué par la gomme xanthane, la gomme guar, la gomme de Tara ou de Caroube, la gomme d'acacia, le carraghénane, l'alginate, le chitosane, la pectine, l'acide citrique, l'acide tartrique, l'acide oxalique, l'acide succinique, l'acide malique, l'acide acétique, l'acide lactique, l'acide propionique, l'acide salicylique, les glycosaminoglycanes. Avantageusement, le phyllosilicate naturel modifié comprend une bentonite modifiée par la gomme de xanthane et l'acide citrique.

Selon un autre mode de réalisation, le phyllosilicate naturel modifié comprend un composé organique choisi parmi tout composé organique connu dans l'art des émulsions de Pickering, tels que ceux divulgués dans le brevet français n° FR 2 976 503.

Avantageusement, le phyllosilicate modifié est présent en une quantité allant de 2% à 20%, de préférence 2,5% à 10% et de manière préférée 3,5% à 8% en poids par rapport au poids total de la composition.

Avantageusement, la phase huileuse de l'émulsion de Pickering est présente en une quantité allant de 5% à 45%, de préférence 10% à 35% et de manière préférée 15% à 30% en poids par rapport au poids total de la composition.

De manière avantageuse, l'émulsion de Pickering H/E est préparée selon un procédé qui comprend au moins les étapes suivantes dans cet ordre :
- préparer la phase aqueuse ;
- ajouter le phyllosilicate organomodifié à la phase aqueuse et mélanger ;
- ajouter la phase huileuse végétale au mélange obtenu et obtenir une émulsion de Pickering H/E.

Avantageusement la composition selon la présente invention comprend au moins un agent cosmétique choisi parmi les agents humectants, les épaississants, les agents de texture, les émulsifiants, les agents dispersants, les agents moussants, les émollients, les conservateurs, les colorants, les extraits de plantes, les fibres végétales, les minéraux, les agents correcteurs de pH, les principes actifs et les parfums.

Avantageusement la composition selon la présente invention comprend de 0,001 à 20 % en poids d'au moins un agent cosmétique par rapport au poids total de la composition.

Bien entendu, l'homme du métier veillera à choisir ces agents cosmétiques de manière à ne pas altérer les propriétés de la composition selon l'invention.

La composition selon l'invention peut constituer une composition de massage, de soin de la peau, et notamment une crème de nettoyage, de protection, de traitement ou de soin pour le visage, pour les mains, pour les pieds, pour le corps, pour le cuir chevelu ou pour les ongles. En particulier la composition peut être une crème de soin, crème de jour, crème de nuit, crème démaquillante, crème de fond de teint ; un fond de teint fluide, un lait de démaquillage, un lait corporel de protection ou de soin ; une lotion, gel ou mousse pour le soin ou l'hygiène de la peau ou du cuir chevelu ou des ongles, comme une lotion micellaire de nettoyage.

### Utilisations

En particulier, la présente invention vise l'utilisation cosmétique, non thérapeutique, par voie topique dudit extrait ou de ladite composition pour ses effets anti-âge, antiride, sur l'éclat du teint et/ou anti-tache.

Plus précisément, la présente invention vise l'utilisation cosmétique, non thérapeutique, par voie topique dudit extrait ou de ladite composition pour effacer les taches d'hyperpigmentation de la peau et/ou améliorer l'éclat de la peau.

En particulier, les taches d'hyperpigmentation de la peau correspondent à l'accumulation de mélanine à la surface de la peau ; l'amélioration de l'éclat de la peau correspond à l'élimination des peaux mortes qui diffractent la lumière en donnant un effet terne à la surface de la peau.

La présente invention vise aussi l'utilisation cosmétique, non thérapeutique, par voie topique dudit extrait ou de ladite composition pour conférer à la peau un aspect plus lisse, plus clair et/ou plus uniforme.

Parmi les propriétés et avantages de la composition selon la présente invention, on compte :
- l'élimination des rides et ridules *via* le lissage du microrelief cutané,
- l'élimination de la corne, c'est-à -dire des callosités saillantes, des mains et des pieds ;
- l'élimination des taches pigmentaires sans affecter la pilosité, ni dégrader la kératine des poils, des cheveux et des ongles.

En particulier les propriétés exfoliantes de la composition selon la présente invention permettent d'éliminer les peaux mortes autour des ongles sans affecter les ongles, d'éliminer les peaux mortes de la peau et du cuir chevelu (pellicules), et ce faisant elle permet d'illuminer le teint (élimine l'aspect terne conféré par les peaux mortes) et/ou d'assainir le cuir chevelu.

Les propriétés exfoliantes de la composition selon la présente invention permettent aussi d'éclaircir le teint en libérant l'accumulation de la mélanine au niveau des cornéocytes, d'assainir la peau par nettoyage et élimination des peaux mortes (substrat nutritif pour les microorganismes), de stimuler la régénération cellulaire de la peau, et d'obtenir une peau fraîche d'apparence plus jeune.

La composition selon la présente invention permet aussi de stimuler la production de collagène et d'élastine : elle permet un renouvellement cellulaire plus rapide et une stimulation de la couche basale de l'épiderme. Cette stimulation induit une production accrue de collagène, d'élastine et des protéines structurelles de la peau, production qui confère à la peau fermeté et élasticité.

Les exemples qui suivent visent à illustrer l'invention sans en limiter la portée.

### Exemples

### A- Exemple 1.1- Extrait de coques de noix de cajou

### Préparation de l'extrait

Les coques de noix de cajou ont été découpées mécaniquement au moyen d'un broyeur à couteaux de manière à obtenir des particules de coques inférieures ou égales à 5 mm.

100 g de coques de noix de cajou découpées ont ensuite été introduites dans un réacteur avec 300 g d'éthanol à 96°.

Le cisaillement a ensuite été maintenu sous agitateur Misceo 4 couteaux pendant 2 heures à une température d'environ 40°C. La température du mélange ne doit pas dépasser 45°C notamment afin d'éviter l'altération de l'acide anacardique en cardanol.

A l'issue de ces 2 heures, le mélange a été filtré sur filtre plissé.

243,55 g de filtrat ont été obtenu, soit un rendement de 60,89%. L'extrait obtenu (filtrat) comprend un taux de matières sèches de 11,17% soit 27,20 g.

Puis le filtrat a été placé dans un rotavapor à 50°C où l'évaporation de l'éthanol a été effectuée. Après évaporation de l'éthanol (206,0g), 4,6 g d'extrait de coques de noix de cajou concentré ont été obtenus.

### Détermination de la composition de l'extrait

Par analyse en chromatographie en phase gazeuse, il a pu être montré que l'extrait obtenu comprend 73% d'acide 6- [8(Z)-n-pentadecenyl] salicylique, correspondant à la formule I avec CH₂R égal C15 :1.

### B- Exemple 1.2 Etude histologique

Une étude sur des lames de peau humaine exposées à un extrait de coque de noix de cajou selon l'invention, avec analyse histologique après coloration HES standard a été réalisée.

Dans le tableau 1 est présenté le gel - exemple 1.2- comprenant une émulsion de Pickering H/E utilisé dans l'étude histologique.

**[Tableau 1]**

| Phase | Nom commercial | % | INCI |
|---|---|---|---|
| A | Eau | Qsp | Aqua |
| A | Benzoate de Sodium | 0,40 | Sodium benzoate |
| B | Frametime LTX commercialisé par Ephyla | 3,5 | Sodium Magnesium Silicate & xanthan gum & citric acid |
| C | Extrait de coques de cajou concentré obtenu à l'exemple 1 | 30,00 | *Anacardium occidentale L* (Cashew shell) extract |
| Total | | 100 | |

Cette émulsion est obtenue en solubilisant le conservateur « Benzoate de sodium » dans l'eau, puis en mixant sous cisaillement à 4500 tours/ minute (rpm) avec un rotor-stator, durant 7 minutes, le Frametime LTX de sorte à obtenir un gel visqueux, enfin l'extrait obtenu selon l'exemple 1.1 (d'affinité huileuse) est incorporé toujours sous cisaillement à 3500 rpm durant 5 minutes pour former une émulsion de Pickering dans laquelle l'extrait représente la phase dispersée.

Le témoin correspond à une lame de peau humaine traitée avec 500 mg de sérum physiologique. Les 4 autres échantillons, traités et évalués à des durées différentes, correspondent à une lame de peau humaine traitée avec 500 mg de gel (exemple 1.2).

Cinq points temporels ont été évalués : témoin (non traité), échantillon traité évalué à 30 min, échantillon traité évalué à 60 min, échantillon traité évalué à 120 min et échantillon traité évalué à 240 min.

L'ensemble des structures dermiques et épidermiques a été évalué.

Le tableau 2 présente l'évolution des altérations cutanées observées.

**[Tableau 2]**

| Temps | Observations Épiderme | Observations Derme |
|---|---|---|
| Témoin | Normal, aucun signe particulier | Cellules inflammatoires normales |
| Echantillon à 30 min | Identique au témoin | Pas de modification |
| Echantillon à 60 min | Épiderme reste normal | légère élastose superficielle possible |
| Echantillon à 120 min | Noyaux altérés vacuolaires | légère élastose superficielle possible |
| Echantillon à 240 min | Détachement de cellules épidermiques, couche cornée intacte | Derme plus dense |

Le rapport a mis en évidence qu'au bout de 4 heures (240 min), l'échantillon traité présente un détachement (exfoliation) des cellules de l'épiderme, sans exfoliation des cellules du derme (absence d'atteinte dermique nécrotique).

Cette étude atteste d'une exfoliation ciblée, limitée à l'épiderme.

### B-Exemple 2-Compositions cosmétiques

### Exemple 2.1

Dans le tableau 3 est présentée une crème selon l'invention comprenant une émulsion de Pickering H/E.

**[Tableau 3]**

| Phase | Nom commercial | % | INCI |
|---|---|---|---|
| A | Eau | Qsp | Aqua |
| A | Benzoate de Sodium | 0,40 | Sodium benzoate |
| B | Frametime CXG commercialisé par Ephyla | 5,0 | Bentonite & xanthan gum & Sodium stearoyl glutamate & citric acid |
| B | Amisoft HS-11P | 0,60 | Sodium stearoyl glutamate |
| B | Gomme de xanthane FF commercialisée par Jungbunzlauer | 0,10 | xanthan gum |
| C | Huile de tournesol raffinée commercialisée par CAUVIN | 25,00 | Helianthus annuus seed oil |
| D | Extrait de coques de cajou concentré obtenu à l'exemple 1.1 | 1,00 | *Anacardium occidentale L* (Cashew shell) extract |
| Total | | 100 | |

D'une part, les constituants des phases A et B ont été mélangés à 20°C avec un mixeur mécanique à couteaux ou un rotor stator à une vitesse comprise entre 3500 et 4000 rpm (tours par minutes) durant 10 minutes pour obtenir un mélange homogénéisé. D'autre part, les constituants des phases C et D ont été mélangés à 20°C sous agitation lente avec une simple hélice. Enfin les deux mélanges obtenus ont été réunis et mélangés avec un mixeur mécanique à couteaux ou un rotor stator à une vitesse de 4000 rpm durant 10 minutes pour obtenir une composition homogénéisée.

Le pH de la composition obtenue est ajusté à 5.

La composition a ensuite été utilisée en application non rincée (ou « leave on »).

Cette composition a été appliquée le matin sur le corps ou visage.

Après environ trois semaines d'utilisation les testeurs notent une tonalité de peau plus claire.

Sur cette même période, les testeurs notent également un effacement des taches d'hyperpigmentation conduisant à un éclaircissement de la surface de la peau.

Par ailleurs, cette composition a été appliquée le soir (la veille du shampooing) sur le cuir chevelu 3 fois par semaine durant 3 semaines.

Dès la première semaine d'utilisation les testeurs notent une disparition des démangeaisons et des desquamations (pellicules) du cuir chevelu.

La composition assainit le cuir chevelu et réduit les pellicules.

### Exemple 2.2

Dans le tableau 4 est présentée une crème selon l'invention.

**[Tableau 4]**

| Phase | Nom commercial | % | INCI |
|---|---|---|---|
| A | Eau | Qsp | Aqua |
| B | Glycérine | 3,50 | glycerin |
| B | Gomme de xanthane FF | 0,10 | xanthan gum |
| | commercialisée par Jungbunzlauer | | |
| C | Cutina GMS | 4,50 | Glyceryl monostearate |
| C | Alcool cétéarylique | 1,00 | Ceterayl alcohol |
| C | Amisoft HS-11P | 0,30 | Sodium stearoyl glutamate |
| C | Huile de tournesol raffinée commercialisée par CAUVIN | 25,00 | Helianthus annuus seed oil |
| D | Extrait de coques de cajou concentré obtenu à l'exemple 1.1 | 0,25 | *Anacardium occidentale L* (Cashew shell) extract |
| E | Acide citrique monohydraté | 0,40 | Citric acid |
| Total | | 100 | |

D'une part, la phase B a été mélangée à l'hélice, à température ambiante, jusqu'à solubilisation de la gomme de xanthane.

D'autre part la phase C a été portée à 80°C pour la faire fondre.

Ensuite la phase A a été chauffée à 45°C, et la phase C, toujours maintenue à 80°C, a été ajoutée dans la phase A.

Le mélange des phases (A et C) a ensuite été émulsionné avec la phase B au moyen d'un mixer ou un rotor-stator avec une vitesse de 3000 à 4500 rpm jusqu'à homogénéisation, environ 7 minutes.

L'émulsion a ensuite été refroidie à 40°C et la phase D a été ajoutée sous agitation lente avec une simple hélice ; enfin la phase E a été ajoutée avec un rotor-stator à une vitesse de 3000 à 4500 rpm durant 5 minutes.

Le pH de la composition obtenue est ajusté à 5.

La composition est utilisée en application non rincée (ou « leave on »).

Cette composition a été appliquée le matin et le soir sur le corps ou visage.

Après environ trois semaines d'utilisation les testeurs notent une tonalité de peau plus claire.

Sur cette même période, les testeurs notent également un effacement des taches d'hyperpigmentation conduisant à un éclaircissement de la surface de la peau.

Par ailleurs, cette composition a été appliquée le soir (la veille du shampooing) sur le cuir chevelu 3 fois par semaine durant 3 semaines.

Après cette période d'utilisation les testeurs notent une disparition des démangeaisons et des desquamations (pellicules) du cuir chevelu.

La composition assainit le cuir chevelu et réduit les pellicules.

### Exemple 2.3

Dans le tableau 5 est présentée un masque gommage « peeling » corps et visage selon l'invention comprenant une émulsion de Pickering H/E.

**[Tableau 5]**

| Phase | Nom commercial | % | INCI |
|---|---|---|---|
| A | Eau | Qsp | Aqua |
| A | Frametime CX commercialisé par Ephyla | 4,50 | Bentonite & xanthan gum & citric acid |
| A | Gomme de xanthane FF | 0,10 | xanthan gum |
| | commercialisée par Jungbunzlauer | | |
| B | Huile de tournesol raffinée commercialisée par CAUVIN | 25,00 | Helianthus annuus seed oil |
| B | Extrait de coques de cajou concentré obtenu à l'exemple 1.1 | 30,00 | *Anacardium occidentale L* (Cashew shell) extract |
| C | Novasolve DMI | 3,00 | Dimethyl isosorbide |
| C | Propanediol | 6,00 | Propanediol |
| C | Sorbitol | 0,80 | Sorbitol |
| Total | | 100 | |

Les constituants de la phase A ont été mélangés à 20°C avec un mixeur mécanique à couteaux ou un rotor stator à une vitesse comprise entre 3500 et 4000 rpm jusqu'à homogénéisation, environ 7 minutes.

Puis la phase A a été mélangée avec les constituants de la phase B à 20°C avec un mixeur mécanique à couteaux ou un rotor stator à une vitesse comprise entre 3500 et 4000 rpm jusqu'à homogénéisation, environ 5-7 minutes.

Enfin la phase a été ajoutée à 20°C sous agitation lente avec un mixeur mécanique à couteaux ou un rotor stator à une vitesse comprise entre 2000 et 2500 rpm pendant 4 à 5 minutes.

Le pH de la composition obtenue est ajusté à 6,5-7,5.

Cette composition de masque a été appliquée en couche épaisse pendant 10 à 60 minutes puis elle a été rincée à l'eau claire.

Sur le visage, on a observé dès la première application, un effet peeling des cornéocytes c'est-à-dire une abrasion du relief cutané et une tonalité de peau plus claire.

Sur le visage les testeurs notent, dès la première application, un effacement des taches d'hyperpigmentation conduisant à un éclaircissement de la surface de la peau.

Sur le tour des ongles, le produit est appliqué en couche épaisse entre 30 et 45 minutes, puis rincé à l'eau claire, les peaux mortes disparaissent au rinçage en frottant légèrement avec une spatule de bois ; le pourtour des ongles est d'apparence très propre et net sans peau intermédiaire.

Sur la face externe des talons de pieds, au niveau des zones de « corne », le produit est appliqué en couche épaisse entre 30 et 45 minutes, puis rincé à l'eau claire, la « corne » disparait au rinçage en frottant légèrement avec une spatule de bois ; la corne a laissé place à une peau souple et beaucoup plus tendre.

La composition selon l'invention procure un effet anti-âge par lissage dès la première application.

### Exemple 2.4

Dans le tableau 6 est présenté un masque gommage « peeling leave on » corps et visage selon l'invention sans phase aqueuse.

**[Tableau 6]**

| Phase | Nom commercial | % | INCI |
|---|---|---|---|
| A | OLO3D commercialisé par Ephyla | 14,00 | Stearalkonium Bentonite |
| B | Huile de tournesol raffinée | 57,00 | Helianthus annuus seed oil |
| | commercialisée par CAUVIN | | |
| B | Huile de moringa commercialisée par Ephyla | 5,00 | Moringa oleifera seed oil |
| B | Huile biologique de dattier du désert commercialisée par Ephyla | 5,00 | Balanites roxburghii seed oil |
| B | HTRE commercialisée par Ephyla | 3,00 | Helianthus annuus seed oil & Canarium luzonicum gum non volatiles |
| C | Extrait de coques de cajou concentré obtenu à l'exemple 1.1 | 16,00 | *Anacardium occidentale L* (Cashew shell) extract |
| Total | | 100 | |

Les constituants des phases A et B ont été mélangés à 20°C avec un mixeur mécanique à couteaux ou un rotor stator à une vitesse comprise entre 3500 et 4000 rpm durant 7 minutes pour obtenir un mélange homogénéisé. Puis la phase C a été ajoutée à 20°C et mélangée avec un mixeur mécanique à couteaux ou un rotor stator à une vitesse comprise entre 3500 et 4000 rpm pour obtenir une composition homogénéisée (environ 5-7 minutes).

Cette composition a été utilisée en application non rincée (ou « leave on ») directement sur les taches afin de cibler les taches.

On a observé un effet pulpant qui lisse la surface de la peau et apporte un bel éclat du teint. La composition selon l'invention procure un effet anti-âge par lissage.

### Exemple 2.5

Dans le tableau 7 est présentée un sérum de nuit selon l'invention comprenant une émulsion de Pickering H/E.

**[Tableau 7]**

| Phase | Nom commercial | % | INCI |
|---|---|---|---|
| A | Eau | Qsp | Aqua |
| A | Glycérine | 3,00 | glycerin |
| A | Benzoate de Sodium | 0,40 | Sodium benzoate |
| B | Frametime CXG commercialisé par Ephyla | 5,0 | Bentonite & xanthan gum & Sodium stearoyl glutamate & citric acid |
| B | Amisoft HS-11P | 0,60 | Sodium stearoyl glutamate |
| B | Gommme de xanthane FF commercialisée par Jungbunzlauer | 0,15 | xanthan gum |
| C | HTRE commercialisée par Ephyla | 2,50 | Helianthus annuus seed oil & Canarium luzonicum gum non volatiles |
| C | Huile de tournesol raffinée commercialisée par CAUVIN | 12,50 | Helianthus annuus seed oil |
| D | Extrait de coques de cajou | 10,00 | *Anacardium occidentale L* (Cashew shell) extract |
| | concentré obtenu à l'exemple 1.1 | | |
| E | Acide citrique monohydraté | 0,40 | Citric acid |
| Total | | 100 | |

D'une part, les constituants des phases A et B ont été mélangés à 20°C avec un mixeur mécanique à couteaux ou un rotor stator à une vitesse comprise entre 3500 et 4000 rpm durant 7 minutes pour obtenir un mélange homogénéisé. D'autre part, les constituants des phases C et D ont été mélangés à 20°C sous agitation lente avec une simple hélice. Enfin les deux mélanges obtenus ont été réunis et mélangés avec un mixeur mécanique à couteaux ou un rotor stator à une vitesse comprise entre 3000 et 4500 jusqu'à homogénéisation, 5-7 minutes.

Le pH de la composition obtenue a été ajusté à 5 au moyen de la phase E.

Cette composition a été appliquée en couche fine après un nettoyage de la peau à l'eau claire, puis laissée durant toute la nuit jusqu'au nettoyage du matin à l'eau claire également.

On a observé un effet peeling des cornéocytes c'est-à-dire une abrasion du relief cutanée et une tonalité de peau plus claire sur une semaine d'application le soir.

Les testeurs notent un effacement des taches d'hyperpigmentation conduisant à un éclaircissement de la surface de la peau sur une semaine d'application le soir.

La composition selon l'invention procure un effet anti-âge par lissage.

Les utilisateurs ont noté un effet exfoliant en surface de la peau (épiderme) notable dès les premières applications et notamment des propriétés anti-tache pigmentaires.

## Revendications

1. Utilisation cosmétique, non thérapeutique, par voie topique d'un extrait de coques de noix de cajou ou *« Anacardium occidentale L.* » en tant qu'agent exfoliant de l'épiderme, ledit extrait de coques de noix de cajou étant susceptible d'être obtenu par un procédé de préparation comprenant au moins les étapes suivantes, dans cet ordre :
- fournir des coques de noix de cajou,
- broyer les coques de noix de cajou pour obtenir une taille moyenne des particules inférieure ou égale 5 mm,
- procéder à l'extraction des coques de noix de cajou au moyen d'éthanol à 96° à une température comprise entre 20°C et 45°C,
- filtrer,
- récupérer le filtrat,
- évaporer l'éthanol,
- récupérer l'extrait obtenu.

2. Utilisation selon la revendication 1 dans laquelle l'extrait de coques de noix de cajou comprend de 60 à 95 % en poids d'acide anacardique par rapport au poids total de l'extrait.

3. Utilisation selon la revendication 1 ou 2 dans laquelle l'extrait de coques de noix de cajou comprend de 50 à 90 % en poids d'acide 6-[8(Z)-n-pentadecenyl] salicylique par rapport au poids total de l'extrait.

4. Utilisation selon l'une quelconque des revendications précédentes d'une composition comprenant de 0,1 à 49,9 % en poids de l'extrait de coques de noix par rapport au poids total de la composition.

5. Utilisation selon la revendication 4 dans laquelle la composition comprend de 0,1 à 99,9% en poids d'une phase huileuse végétale comprenant au moins une huile végétale et/ou au moins un ester de corps gras végétal par rapport au poids total de la composition.

6. Utilisation selon la revendication 5 dans laquelle la phase huileuse végétale comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de chanvre, l'huile de nigelle, l'huile de figue de barbarie, l'huile de macadamia, l'huile de soja, l'huile de palme et de palmier, l'huile de Tamanu, l'huile de Kukui, l'huile de sésame, l'huile de lin, l'huile de noix, l'huile de noisette, l'huile d'amande, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan et les triglycérides notamment d'acides caprylique/caprique.

7. Utilisation selon l'une quelconque des revendications 4 à 6 dans laquelle ladite composition comprend une émulsion de Pickering H/E contenant une phase aqueuse, une phase huileuse végétale et un phyllosilicate organomodifié.

8. Utilisation selon la revendication 7 dans laquelle le phyllosilicate organomodifié est présent en une quantité allant de 2% à 20%, de préférence 2,5% à 10% et de manière préférée 3,5% à 8% en poids par rapport au poids total de la composition.

9. Utilisation selon l'une quelconque des revendications 4 à 8 comprenant au moins un agent cosmétique choisi parmi les agents humectants, les épaississants, les agents de texture, les émulsifiants, les agents dispersants, les agents moussants, les émollients, les conservateurs, les colorants, les extraits de plantes, les fibres végétales, les minéraux, les agents correcteurs de pH, les principes actifs et les parfums.

10. Utilisation selon l'une quelconque des revendications précédentes pour effacer les taches d'hyperpigmentation de la peau et/ou améliorer l'éclat de la peau.

11. Utilisation selon la revendication 10our conférer à la peau un aspect plus lisse, plus clair et/ou plus uniforme.

12. Procédé de traitement cosmétique, non thérapeutique d'exfoliation de l'épiderme comprenant au moins une étape d'application sur la peau d'un extrait tel que défini dans l'une quelconque des revendications 1 à 3 ou d'une composition telle que définie dans l'une quelconque des revendications 4 à 9..[ ]
